# EUROPEAN PATENT SPECIFICATION

(11) **EP 3 322 480 B1**
(45) Date of publication and mention of the grant of the patent: **14.10.2020**
(21) Application number: 16736860.4
(22) Date of filing: 07.07.2016
(51) Int. Cl.: A61K 35/747, A61P 9/10, A61K 31/00, A61K 33/00, A61K 35/74, A23L 33/135

(54) **LACTOBACILLI FOR TREATING CARDIAC DYSFUNCTION**
LAKTOBAZILLEN ZUR BEHANDLUNG VON KARDIALER DYSFUNKTION
UTILISATION DE LACTOBACILLI POUR TRAITER UN DYSFONCTIONNEMENT CARDIAQUE

(30) Priority: 16.07.2015 US 201562193539 P; 12.08.2015 GB 201514302
(43) Date of publication of application: 23.05.2018
(73) Proprietor: DuPont Nutrition Biosciences ApS, 1411 Copenhagen K (DK)
(72) Inventor: STENMAN, Lotta, 02460 Kantvik (FI); LAHTINEN, Sampo, 08350 Lohja (FI); KONHILAS, John, Tucson, AZ 85719 (US)
(74) Representative: DuPont EMEA
(86) International application number: PCT/EP2016/066185
(87) International publication number: WO 2017/009188

(56) References cited:
- WO-A1-03/010298
- US-A1- 2006 088 514
- BERGER U ET AL: "[Endocarditis lenta caused by Lactobacillus salivarius subsp. salicinicus (author's transl)]", DEUTSCHE MEDIZINISCHE WOCHENSCHRIFT, DMW DEUTSCHE MEDIZINISCHE WOCHENSCHRIFT FEBRUARY 11, 2005, vol. 101, no. 37, 10 September 1976 (1976-09-10), pages 1349-1350, XP009191472, ISSN: 0012-0472
- LARSEN NADJA ET AL: "Effect of Lactobacillus salivarius Ls-33 on fecal microbiota in obese adolescents", CLINICAL NUTRITION, vol. 32, no. 6, 13 February 2013 (2013-02-13), pages 935-940, XP028776191, ISSN: 0261-5614, DOI: 10.1016/J.CLNU.2013.02.007
- W. S. HARTY ET AL: "The aggregation of human platelets by Lactobacillus species", JOURNAL OF GENERAL MICROBIOLOGY, vol. 139, no. 12, 1 December 1993 (1993-12-01), pages 2945-2951, XP55297689, GB ISSN: 0022-1287, DOI: 10.1099/00221287-139-12-2945
- SLOVER ET AL: "Lactobacillus: a Review", CLINICAL MICROBIOLOGY NEWSLETTER, ELSEVIER, NEW YORK, NY, US, vol. 30, no. 4, 5 February 2008 (2008-02-05), pages 23-27, XP022511490, ISSN: 0196-4399, DOI: 10.1016/J.CLINMICNEWS.2008.01.006

## Description

### Field of the Invention

This invention relates to the use of a bacterium of the species *Lactobacillus salivarius* strain 33 (Ls-33) for treating a number of cardiac conditions, in particular myocardial infarction.

### Background to the Invention

As cardiovascular disease (CVD) remains the leading cause of death in industrialized countries (30% of all global deaths; ref. WHO Fact sheet number 317, Cardiovascular diseases) with 45% of these deaths due to coronary heart disease. Acute coronary events (ACEs) such as myocardial infarction (MI) and/or sudden cardiac death often result from atherosclerotic plaque rupture and the last 15-20 years of research has established a mechanistic link between inflammation in every aspect of the atherosclerotic process including plaque development, rupture and subsequent ACE (Shah PK. Inflammation and plaque vulnerability. Cardiovasc Drugs Ther 2009; 23: 31-40).

Atherosclerosis is the condition in which an artery wall thickens as the result of a build up of fatty materials such as cholesterol. It is a syndrome affecting arterial blood vessels. It is a chronic inflammatory response in the walls of arteries, in large part due to the accumulation of macrophage white blood cells and promoted by low density (especially small particle) lipoproteins (plasma proteins that carry cholesterol and triglycerides) without adequate removal of fats and cholesterol from the macrophages by functional high density lipoproteins (HDL). It is commonly referred to as a hardening or furring of the arteries. It is caused by the formation of multiple plaques within the arteries.

Heart failure is a global term for the physiological state in which cardiac output is insufficient for the body's needs. This may occur when the cardiac output is low (often termed "congestive heart failure"). Common causes of heart failure include myocardial infarction and other forms of ischemic heart disease, hypertension, valvular heart disease and cardiomyopathy.

Myocardial infarction, commonly known as a heart attack, occurs when the blood supply to part of the heart is interrupted causing some heart cells to die. This is most commonly due to occlusion (blockage) of a coronary artery following the rupture of a vulnerable atherosclerotic plaque, which is an unstable collection of lipids (like cholesterol) and white blood cells (especially macrophages) in the wall of an artery. The resulting ischemia (restriction in blood supply) and oxygen shortage, if left untreated for a sufficient period of time, can cause damage and/or death (infarction) of heart muscle tissue (myocardium).

Myocardial infarction may lead to dilated cardiomyopathy in which the heart becomes enlarged and cannot efficiently pump blood. Cardiac tissue contains two major types of collagen: collagen type I (Col I) and type III (Col III). Col I is predominantly present in strong and stiff tissues such as tendons, while Col III is found in more elastic tissues such as skin. In patients with dilated cardiomyopathy, the ratio of Col I expression to Col III is increased in those with more severely impaired cardiac function (smaller ejection fraction) (Pauschinger et al. Circulation 1999; 99(21): 2750-6, Soufen et al. Braz J Med Biol Res 2008; 41(12): 1098-1104).

There is a continuous and on-going hunt for novel and efficacious treatment strategies to minimize CVD development and clinical outcomes.

WO 2009/153662 describes the use of a bacterium selected from a lactic acid bacterium, a *Bifidobacterium* or a mixture of any thereof in the manufacture of a food product, dietary supplement or medicament for treating a number of conditions, including cardiovascular disease.

WO 2010/146568 describes the use of a *Bifidobacterium* or a mixture thereof in the manufacture of a food product, dietary supplement or medicament for treating a number of conditions, including cardiovascular disease.

WO 2013/032538 describes a method for identifying and treating cardiac defects by monitoring and altering the microbiome of the patient. The microbiome may be altered by administration of a number of probiotic bacteria, including various strains of *Lactobacillus* or *Bifidobacterium.*

V. Lam et al. FASEB J. 2012, 26, 1-9 describes the administration of the probiotic *Lactobacillus plantarum* 299v and its effects on myocardial infarction in rats.

However, none of the prior art documents specifically disclose the use of a bacterium of the species *Lactobacillus salivarius,* in particular the specific strain 33 of *Lactobacillus salivarius* (Ls-33) to treat myocardial infarction, dilated cardiomyopathy, or other forms of cardiac dysfunction.

### Summary of the Invention

In one aspect, the invention provides a bacterium of the species *Lactobacillus salivarius* strain 33 (Ls-33) (PTA-4800), for use in treating cardiac dysfunction in a mammal.

In particular, the invention provides a bacterium of the species *Lactobacillus salivarius* strain 33 (Ls-33) (PTA-4800), for use in treating myocardial infarction in a mammal.

In particular, the invention provides a bacterium of the species *Lactobacillus salivarius* strain 33 (Ls-33) (PTA-4800), for use in treating dilated cardiomyopathy in a mammal.

In particular, the invention provides a bacterium of the species *Lactobacillus salivarius* strain 33 (Ls-33) (PTA-4800), for use in treating congestive heart failure in a mammal.

In particular, the invention provides a bacterium of the species *Lactobacillus salivarius* strain 33 (Ls-33) (PTA-4800), for use in treating inflammatory heart disease in a mammal.

In some aspects, the treatment of cardiac dysfunction (e.g. myocardial infarction, dilated cardiomyopathy, congestive heart failure or inflammatory heart disease) comprises lowering of the collagen I : collagen III expression ratio in the mammal.

Therefore, in a further aspect, the invention provides a bacterium of the species *Lactobacillus salivarius* strain 33 (Ls-33) (PTA-4800) for use in lowering of the collagen I : collagen III expression ratio in a mammal.

### Advantages

It has surprisingly been found by the present inventors that treatment with a probiotic *Lactobacillus salivarius* strain 33 (Ls-33) in a model of ischemia-reperfusion, which simulates myocardial infarction, causes decreased expression of Col I compared to Col III. This confers the potential for Ls-33 to be useful in treating a number of cardiovascular diseases, in particular myocardial infarction. In particular, lowering of the collagen I : collagen III expression ratio may contribute to reducing or alleviating the consequences of a myocardial infarction and in preventing dilated cardiomyopathy.

### Brief Description of Drawings

Figure 1 shows the Col I/Col III ratio in both ischemic (MI) and non-ischemic (non-MI) regions of the heart in mice on a high-fat diet treated with Ls-33; and
Figure 2 shows the Col I/Col III ratio in both ischemic (MI) and non-ischemic (non-MI) regions of the heart in mice on a normal-fat diet treated with Ls-33.

### Detailed Description

### Bacteria

The bacterium used in the present invention is a bacterium of the species *Lactobacillus salivarius.* Preferably, the bacterium used in the present invention is a probiotic strain of the species *Lactobacillus salivarius.* In this specification the term probiotic strain' is defined as covering a non-pathogenic strain of the bacterium which, when administered live in adequate amounts, confer a health benefit on the host. These probiotic strains generally have the ability to survive the passage through the upper part of the digestive tract. They are non-pathogenic, non-toxic and exercise their beneficial effect on health on the one hand via ecological interactions with the resident flora in the digestive tract, and on the other hand via their ability to influence the immune system in a positive manner via the "GALT" (gut-associated lymphoid tissue). Depending on the definition of probiotics, these bacteria, when given in a sufficient number, have the ability to progress live through the intestine, however they do not cross the intestinal barrier and their primary effects are therefore induced in the lumen and/or the wall of the gastrointestinal tract. They then form part of the resident flora during the administration period. This colonization (or transient colonization) allows the probiotic bacteria to exercise a beneficial effect, such as the repression of potentially pathogenic micro-organisms present in the flora and interactions with the immune system of the intestine.

Examples of probiotic strain of the species *Lactobacillus salivarius* include *Lactobacillus salivarius* strain 33 (Ls-33), described below, *Lactobacillus salivarius* strain UCC118 (Dunne C. et al, Antonie Van Leeuwenhoek 1999, 76(1-4), 279-292)., *Lactobacillus salivarius* strain WB21 (T. Iwamoto et al, Oral Surg Oral Med Oral Pathol Oral Radiol Endod. 2010 110(2), 201-8), *Lactobacillus salivarius* strain TI 2711 (Nishihara T, et al. BMC Oral Health 2014;14:110), *Lactobacillus salivarius* strain UBL S22 (Rajkumar H, et al. J Cardiovasc Pharmacol Ther 2014;E-pub ahead of print Oct 20^{th} 2014), *Lactobacillus salivarius* strain LS01 (Niccoli L, et al. J Clin Gastroenterol 2014;48(Suppl 1):S34-6) and *Lactobacillus salivarius* strain MTC 1026 (S. Tinrat et al. J Gen Appl Microbiol. 2011, 57(6) 365-78).

The bacterium used in the present invention is *Lactobacillus salivarius* strain 33 (Ls-33). This strain of *Lactobacillus salivarius* is also known as *Lactobacillus salivarius* PTA-4800. This strain is commercially available from DuPont Nutrition Biosciences ApS. This strain of *Lactobacillus salivarius* has also been deposited by Rhodia Chimie, 26, quai Alphonse Le Gallo, 92 512 Boulogne-Billancourt Cedex, France, in accordance with the Budapest Treaty on 15 November 2002 at the American Type Culture Collection (ATCC) 10801 University Blvd. Manassas, Virginia 20110-2209, United States of America, where it is recorded under registration number PTA-4800.

The bacterium may be used in any form capable of exerting the effects described herein. For example, the bacteria may be viable, dormant, inactivated or dead bacteria. Preferably, the bacteria are viable bacteria.

The bacteria may comprise whole bacteria or may comprise bacterial components. Examples of such components include bacterial cell wall components such as peptidoglycan, bacterial nucleic acids such as DNA and RNA, bacterial membrane components, and bacterial structural components such as proteins, carbohydrates, lipids and combinations of these such as lipoproteins, glycolipids and glycoproteins.

The bacteria may also or alternatively comprise bacterial metabolites. In this specification the term 'bacterial metabolites' includes all molecules produced or modified by the (probiotic) bacteria as a result of bacterial metabolism during growth, survival, persistence, transit or existence of bacteria during probiotic product manufacture and storage and during gastrointestinal transit in a mammal. Examples include all organic acids, inorganic acids, bases, proteins and peptides, enzymes and co-enzymes, amino acids and nucleic acids, carbohydrates, lipids, glycoproteins, lipoproteins, glycolipids, vitamins, all bioactive compounds, metabolites containing an inorganic component, and all small molecules, for example nitrous molecules or molecules containing a sulphurous acid.

Preferably the bacteria comprise whole bacteria, more preferably whole viable bacteria.

In one embodiment, the bacteria of the species *Lactobacillus salivarius* strain 33 (Ls-33) are used in combination with one or more other probiotic bacteria (as defined above).
In one embodiment, the additional probiotic bacterium is a probiotic bacterium of the genus *Lactobacillus.* The *Lactobacillus* bacteria used may be of the same type (species and strain) or may comprise a mixture of species and/or strains. Typically, the *Lactobacillus* bacteria are selected from the species *Lactobacillus acidophilus, Lactobacillus casei, Lactobacillus kefiri, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sakei, Lactobacillus reuteri, Lactobacillus fermentum, Lactobacillus farciminis, Lactobacillus lactis, Lactobacillus delbreuckii, Lactobacillus plantarum, Lactobacillus paraplantarum, Lactobacillus crispatus, Lactobacillus gasseri, Lactobacillus johnsonii* and *Lactobacillus jensenii,* and combinations of any thereof.

In one embodiment, the additional probiotic bacterium is a probiotic bacterium of the genus *Bifidobacterium.* The *Bifidobacteria* used may be of the same type (species and strain) or may comprise a mixture of species and/or strains. Suitable *Bifidobacteria* are selected from the species *Bifidobacterium bifidium, Bifidobacterium longum, Bifidobacterium animalis, Bifidobacterium breve, Bifidobacterium infantis, Bifidobacterium catenulatum, Bifidobacterium pseudocatenulatum, Bifidobacterium adolescentis,* and *Bifidobacterium angulatum,* and combinations of any thereof.

Preferably, the *Bifidobacterium* used as an additional bacterium in the present invention is of the species *Bifidobacterium animalis.* More preferably, the *Bifidobacterium* used in the present invention is of the species *Bifidobacterium animalis* subsp. *lactis.* In a particularly preferred embodiment, the additional bacteria used in the present invention are *Bifidobacterium animalis* subsp. *lactis* strain 420 (B420). This strain is available from DuPont Nutrition Biosciences ApS. This strain of *Bifidobacterium animalis* subsp. *lactis* has also been deposited under the reference DGCC420 by DuPont Nutrition Biosciences ApS, of Langebrogade 1, 1411 Copenhagen K, Denmark, in accordance with the Budapest Treaty on 30 June 2015 at the Leibniz-Institut Deutsche Sammlung von Mikroorganismen und Zellkulturen GmbH (DSMZ), Inhoffenstrasse 7B, 38124 Braunschweig, Germany, where it is recorded under registration number DSM 32073.

Preferably the bacteria to be used in the present invention are bacteria which are generally recognised as safe and, which is preferably GRAS approved.

### Dosage

The bacteria of the species *Lactobacillus salivarius* strain 33 (Ls-33) used in accordance with the present invention may be administered at a dosage of from about 10⁶ to about 10¹² CFU of bacteria/dose, preferably about 10⁷ to about 10¹¹ CFU of bacteria/dose, more preferably about 10⁸ to about 10¹⁰ CFU of bacteria /dose, more preferably about 5 x 10⁸ to about 5 x 10⁹ CFU of bacteria /dose, and most preferably about 8 x 10⁸ to about 2 x 10⁹CFU of bacteria /dose. CFU stands for "colony-forming units".

By the term "per dose" it is meant that this amount of bacteria is provided to a subject either per day or per intake, preferably per day. For example, if the bacteria are to be administered in a food product (for example in a yoghurt) then the food product (e.g. yoghurt) will preferably contain from about 10⁸ to 10¹²CFU of the bacteria, more preferably about 10⁷ to about 10¹¹ CFU, and most preferably about 10⁹ to about 10¹⁰ CFU of the bacteria. If the bacteria are to be administered in a dietary supplement, then the dietary supplement will preferably contain about 10⁸ to about 10¹² CFU, more preferably about 10⁹ to 10¹¹ CFU of the bacteria. If the bacteria are to be administered in a pharmaceutical composition, then the pharmaceutical composition will preferably contain about 10⁸ to about 10¹² CFU, more preferably about 10⁹ to 10¹¹ CFU of the bacteria. Alternatively, however, this amount of bacteria may be split into multiple administrations each consisting of a smaller amount of microbial loading - so long as the overall amount of bacteria received by the subject in any specific time (for instance each 24 hour period) is within the ranges specified above.

The concentration of the bacteria per g of support varies depending on the intended dose and the nature of the support. In one embodiment, the bacteria of the species *Lactobacillus salivarius* strain 33 (Ls-33) used in accordance with the present invention may be present in a concentration of from 5000 to 10¹³CFU of bacteria/ g of support, preferably about 5 x 10⁶ to about 10¹² CFU of bacteria / g of support. When the support is a food product, such as a yogurt, the bacteria are preferably present in a concentration of from about 5000 to about 5 x 10⁸ CFU of bacteria/ g of food product, preferably about 5 x 10⁶ to about 5 x 10⁷ CFU of bacteria / g of food product. When the support is a dietary supplement, the bacteria are preferably present in a concentration of from 10⁹ to about 10¹³ CFU / g of dietary supplement, more preferably about 10¹⁰ to 10¹² CFU / g of dietary supplement. When the support is a pharmaceutical composition, the bacteria are preferably present in a concentration of from 10⁹ to about 10¹³ CFU / g of dietary supplement, more preferably about 10¹⁰ to 10¹² CFU / g of pharmaceutical composition.

In one embodiment, the bacteria of the species *Lactobacillus salivarius* strain 33 (Ls-33) may be administered once per day at a dosage of from about 10⁶ to about 10¹² CFU of bacteria / day, preferably about 10⁸ to about 10¹²CFU of bacteria / day. Hence, the effective amount in this embodiment may be from about 10⁶ to about 10¹² CFU of bacteria /day, preferably about 10⁷ to about 10¹¹ CFU of bacteria / day. For example, if the bacteria are to be administered in a food product (for example in a yoghurt) - then the bacteria will preferably be administered once per day in a dosage of about 10⁶ to 10¹²CFU of bacteria / day, more preferably about 10⁷ to about 10¹¹ CFU of bacteria / day and most preferably about 10⁹ to about 10¹⁰ CFU of the bacteria / day. If the bacteria are to be administered in a dietary supplement, then the bacteria will preferably be administered once per day in a dosage of about 10⁸ to about 10¹² CFU of bacteria / day, more preferably about 10⁹ to 10¹¹ CFU of the bacteria / day. If the bacteria are to be administered in a pharmaceutical composition, then the , then the bacteria will preferably be administered once per day in a dosage of about 10⁸ to about 10¹² CFU bacteria / day, more preferably about 10⁹ to 10¹¹ CFU of the bacteria / day.

When one or more other microorganisms (such as bacteria, preferably probiotic bacteria) are used in combination with the bacteria of the species *Lactobacillus salivarius* strain 33 (Ls-33) in accordance with the present invention), the other microorganisms may similarly be administered at a dosage of from about 10⁶ to about 10¹² CFU of microorganism/dose, preferably about 10⁷ to about 10¹¹ CFU of microorganism/dose. Depending on the manner in which the microorganisms are administered (food product, dietary supplement, or pharmaceutical composition), the preferred amounts of the one or more other microorganisms will typically be in the ranges specified above for *Lactobacillus salivarius,* preferably *Lactobacillus salivarius* strain 33 (Ls-33).

### Effects / Subjects / Medical Indications

As described above, administration of a probiotic *Lactobacillus salivarius* strain 33 (Ls-33) to a subject causes decreased expression of Col I compared to Col III. This confers the potential for probiotic strains of *Lactobacillus salivarius* Ls-33, to be useful in treating a number of cardiovascular diseases, in particular myocardial infarction. In particular, lowering of the Col I : Col III expression ratio may contribute to reducing or alleviating the consequences of a myocardial infarction and in preventing dilated cardiomyopathy.

In one embodiment, the lowering of the Col I : Col III expression ratio comprises reduction of the Col I : Col III expression ratio by 10% to 90%, preferably 30 to 70%, more preferably 40 to 60% compared with a mammal that has not been treated with a probiotic *Lactobacillus salivarius* strain 33 (Ls-33). Such a reduction can particularly be observed in the ischemic tissue of mammals, especially those ingesting a high-fat diet.

In one embodiment, the lowering of the Col I : Col III expression ratio comprises reduction of the Col I : Col III expression ratio by 30% to 99%, preferably 50 to 90%, more preferably 65 to 75% compared with a mammal that has not been treated with a probiotic *Lactobacillus salivarius* strain 33 (Ls-33). Such a reduction can particularly be observed in the non-ischemic tissue of mammals, especially those ingesting a high-fat diet.

In one embodiment, the lowering of the Col I : Col III expression ratio comprises reduction of the Col I : Col III expression ratio by 0.1% to 20%, preferably 0.1 to 10%, more preferably 1 to 5% compared with a mammal that has not been treated with a probiotic *Lactobacillus salivarius* strain 33 (Ls-33). Such a reduction can particularly be observed in the ischemic tissue of mammals, especially those ingesting a normal-fat diet.

In one embodiment, the lowering of the Col I : Col III expression ratio comprises reduction of the Col I : Col III expression ratio by 1% to 20%, preferably 5 to 15%, more preferably 10 to 12% compared with a mammal that has not been treated with a probiotic *Lactobacillus salivarius* strain 33 (Ls-33). Such a reduction can particularly be observed in the non-ischemic tissue of mammals, especially those ingesting a normal-fat diet.

The *Lactobacillus salivarius* strain 33 (Ls-33) (and any additional bacteria, if present) used in the present invention are administered to a mammal, including for example livestock (including cattle, horses, pigs, chickens and sheep), and humans. In some aspects of the present invention the mammal is a companion animal (including pets), such as a dog or a cat for instance. In some aspects of the present invention, the subject may suitably be a human.

The *Lactobacillus salivarius* strain 33 (Ls-33) (and any additional bacteria, if present) used in the present invention relates may be suitable for treating a number of diseases or conditions in mammals (particularly humans). In this specification the term "treatment" or "treating" refers to any administration of the *Lactobacillus salivarius* strain 33 (Ls-33) (and any additional bacteria, if present) according to the present invention and includes: (1) preventing the specified disease from occurring in a mammal which may be predisposed to the disease but does not yet experience or display the pathology or symptomatology of the disease (including prevention of one or more risk factors associated with the disease); (2) inhibiting the disease in a mammal that is experiencing or displaying the pathology or symptomatology of the diseased (*i.e*., arresting further development of the pathology and/or symptomatology), or (3) ameliorating the disease in a mammal that is experiencing or displaying the pathology or symptomatology of the diseased (*i.e.,* reversing the pathology and/or symptomatology).

In particular, the *Lactobacillus salivarius* strain 33 (Ls-33) (and any additional bacteria, if present) is suitable for the treatment of mammals ingesting a high-fat diet. This aspect is discussed in more detail below.

The *Lactobacillus salivarius* strain 33 (Ls-33) (and any additional bacteria, if present) are used according to the present invention for treating cardiac dysfunction in a patient. Examples of cardiac dysfunction treatable by use of the *Lactobacillus salivarius* strain 33 (Ls-33) (and any additional bacteria, if present) according to the present invention include congestive heart failure (CHF), coronary artery disease and myocardial infarction (heart attack), dilated cardiomyopathy , inflammatory heart disease (including endocarditis, inflammatory cardiomegaly and myocarditis) and peripheral vascular disease.

Heart failure is a global term for the physiological state in which cardiac output is insufficient for the body's needs. This may occur when the cardiac output is low (often termed "congestive heart failure"). Common causes of heart failure include myocardial infarction and other forms of ischemic heart disease, hypertension, valvular heart disease and cardiomyopathy.

Coronary disease (or coronary heart disease) refers to the failure of coronary circulation to supply adequate circulation to cardiac muscle and surrounding tissue. It is most commonly equated with atherosclerotic coronary artery disease, but coronary disease can be due to other causes, such as coronary vasospasm. It is possible for the stenosis to be caused by the spasm.

Myocardial infarction, commonly known as a heart attack, occurs when the blood supply to part of the heart is interrupted causing some heart cells to die. This is most commonly due to occlusion (blockage) of a coronary artery following the rupture of a vulnerable atherosclerotic plaque, which is an unstable collection of lipids (like cholesterol) and white blood cells (especially macrophages) in the wall of an artery. The resulting ischemia (restriction in blood supply) and oxygen shortage, if left untreated for a sufficient period of time, can cause damage and/or death (infarction) of heart muscle tissue (myocardium).

Dilated cardiomyopathy is a condition in which the heart becomes enlarged and cannot pump blood efficiently. Dilated cardiomyopathy is the most common form of non-ischemic cardiomyopathy. About one in three cases of congestive heart failure (CHF) is due to dilated cardiomyopathy.

In one embodiment, the cardiac dysfunction is selected from congestive heart failure (CHF), inflammatory heart disease, dilated cardiomyopathy and myocardial infarction. In one embodiment, the cardiac dysfunction is selected from congestive heart failure (CHF), coronary artery disease and myocardial infarction. In a particularly preferred embodiment, the cardiac dysfunction is myocardial infarction.

The *Lactobacillus salivarius* strain 33 (Ls-33) (and any additional bacteria, if present) used in the present invention relates are suitable for treating myocardial infarction in a mammal. In one embodiment the term "treating myocardial infarction" comprises preventing, or reducing the probability of suffering from, myocardial infarction. In one embodiment the term "treating myocardial infarction" comprises reducing the impact of, or the consequences of, myocardial infarction. In one embodiment the term "treating myocardial infarction" comprises reducing the size of the myocardial infarction. In one embodiment the term "treating myocardial infarction" comprises aiding or accelerating the subject's recovery from a myocardial infarction. In each of the above embodiments the improvement is at least 10%, at least 20%, at least 30%, at least 40%, at least 50%, at least 60%, at least 70%, at least 80%, at least 90% in comparison with a subject that has not been treated with *Lactobacillus salivarius* strain 33 (Ls-33) (and any additional bacteria, if present) according to the present invention.

It is envisaged within the scope of the present invention that the embodiments of the invention can be combined such that combinations of any of the features described herein are included within the scope of the present invention. In particular, it is envisaged within the scope of the present invention that any of the therapeutic effects of the bacteria may be exhibited concomitantly.

### Diet

As noted above, subject mammals treated with bacteria according to the present invention may ingest a high-fat diet. In this specification the term high-fat diet' means a diet generally containing at least 20%, preferably at least 25%, such as at least 30%, for example at least 35%, such as at least 40%, for example at least 45%, such as at least 50%, for example at least 55%, such as at least 60%, for example at least 65%, such as at least 70%, for example at least 75%, such as at least 80%, for example at least 85%, such as at least 90% of calories from fat.

In some embodiments, subject mammals treated with bacteria according to the present invention may ingest a normal-fat diet. In this specification the term 'normal-fat diet' means a diet generally containing less than 30%, preferably less than 25%, less than 20%, for example 1 to 20%, such as 5 to 20%, for example 10 to 20%, of calories from fat.

### Compositions

While it is possible to administer *Lactobacillus salivarius* strain Ls-33 (and any additional bacteria, if present) alone according to the present invention (i.e. without any support, diluent or excipient), the *Lactobacillus salivarius* Ls-33 (and any additional bacteria, if present) are typically and preferably administered on or in a support or as part of a composition or product, in particular as a component of a food product, a dietary supplement or a pharmaceutical formulation. These products typically contain additional components well known to those skilled in the art.

Any product which can benefit from the composition may be used in the present invention. These include but are not limited to foods, particularly fruit conserves and dairy foods and dairy food-derived products, and pharmaceutical products. The *Lactobacillus salivarius* strain Ls-33 (and any additional bacteria, if present) may be referred to herein as "the composition of the present invention" or "the composition".

### Food

In one embodiment, the *Lactobacillus salivarius* strain Ls-33 (and any additional bacteria, if present) are employed according to the invention in a food product such as a food supplement, a drink or a powder based on milk. Here, the term "food" is used in a broad sense - and covers food for humans as well as food for animals (i.e. a feed). In a preferred aspect, the food is for human consumption.

The food may be in the form of a solution or as a solid - depending on the use and/or the mode of application and/or the mode of administration.

When used as, or in the preparation of, a food, such as functional food, the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the composition of the present invention can be used as an ingredient to soft drinks, a fruit juice, energy drinks, sports drinks and sports nutritional supplements or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods (including snack bars), balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified and non-fortified coffee beverage.

The composition can further be used as an ingredient in food products such as American cheese sauce, anti-caking agent for grated & shredded cheese, chip dip, cream cheese, dry blended whip topping fat free sour cream, freeze/thaw dairy whipping cream, freeze/thaw stable whipped topping, low fat and light natural cheddar cheese, low fat Swiss style yoghurt, aerated frozen desserts, hard pack ice cream, label friendly, improved economics & indulgence of hard pack ice cream, low fat ice cream: soft serve, barbecue sauce, cheese dip sauce, cottage cheese dressing, dry mix Alfredo sauce, mix cheese sauce, dry mix tomato sauce and others.

The term "dairy product" as used herein is meant to include a medium comprising milk of animal and/or vegetable origin. As milk of animal origin there can be mentioned cow's, sheep's, goat's or buffalo's milk. As milk of vegetable origin there can be mentioned any fermentable substance of vegetable origin which can be used according to the invention, in particular originating from soybeans, rice or cereals.

Still more preferably the food product employed according to the invention is a fermented milk or humanized milk.

For certain aspects, preferably the present invention may be used in connection with yoghurt production, such as fermented yoghurt drink, yoghurt, drinking yoghurt, cheese, fermented cream, milk based desserts and others.

Suitably, the composition can be further used as an ingredient in one or more of cheese applications, meat applications, or applications comprising protective cultures.

The present invention also describes a method of preparing a food or a food ingredient, the method comprising admixing the composition according to the present invention with another food ingredient.

Advantageously, the present invention relates to products that have been contacted with the composition of the present invention (and optionally with other components/ingredients), wherein the composition is used in an amount to be capable of improving the nutrition and/or health benefits of the product.

As used herein the term "contacted" refers to the indirect or direct application of the composition of the present invention to the product. Examples of the application methods which may be used, include, but are not limited to, treating the product in a material comprising the composition, direct application by mixing the composition with the product, spraying the composition onto the product surface or dipping the product into a preparation of the composition.

Where the product of the invention is a foodstuff, the composition of the present invention is preferably admixed with the product. Alternatively, the composition may be included in the emulsion or raw ingredients of a foodstuff. In a further alternative, the composition may be applied as a seasoning, glaze, colorant mixture, and the like. The compositions of the present invention may be applied to intersperse, coat and/or impregnate a product with a controlled amount of a microorganism.

Preferably, the composition is used to ferment milk or sucrose fortified milk or lactic media with sucrose and/or maltose where the resulting media containing all components of the composition - i.e. said microorganism according to the present invention - can be added as an ingredient to yoghurt milk in suitable concentrations - such as for example in concentrations in the final product which offer a daily dose of 10⁶-10¹¹ cfu. The microorganism according to the present invention may be used before or after fermentation of the yoghurt.

For some aspects the microorganisms according to the present invention are used as, or in the preparation of, animal feeds, such as livestock feeds, in particular poultry (such as chicken) feed, or pet food.

Advantageously, where the product is a food product, the *Lactobacillus salivarius* strain Ls-33 (and any additional bacteria, if present) should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Food Ingredient

The composition of the present invention may be used as a food ingredient and/or feed ingredient.

As used herein the term "food ingredient" or "feed ingredient" includes a formulation which is or can be added to functional foods or foodstuffs as a nutritional supplement.

The food ingredient may be in the form of a solution or as a solid - depending on the use and/or the mode of application and/or the mode of administration.

### Food Supplements

The composition of the present invention may be - or may be added to - food supplements (also referred to herein as dietary supplements).

### Functional Foods

The composition of the present invention may be - or may be added to - functional foods. As used herein, the term "functional food" means food which is capable of providing not only a nutritional effect, but is also capable of delivering a further beneficial effect to consumer.

Accordingly, functional foods are ordinary foods that have components or ingredients (such as those described herein) incorporated into them that impart to the food a specific functional - e.g. medical or physiological benefit - other than a purely nutritional effect.

Although there is no legal definition of a functional food, most of the parties with an interest in this area agree that they are foods marketed as having specific health effects beyond basic nutritional effects.

Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

### Medicament

The term "medicament" as used herein encompasses medicaments for both human and animal usage in human and veterinary medicine. In addition, the term "medicament" as used herein means any substance which provides a therapeutic and/or beneficial effect. The term "medicament" as used herein is not necessarily limited to substances which need Marketing Approval, but may include substances which can be used in cosmetics, nutraceuticals, food (including feeds and beverages for example), probiotic cultures, and natural remedies. In addition, the term "medicament" as used herein encompasses a product designed for incorporation in animal feed, for example livestock feed and/or pet food.

### Pharmaceutical

The composition of the present invention may be used as - or in the preparation of - a pharmaceutical. Here, the term "pharmaceutical" is used in a broad sense - and covers pharmaceuticals for humans as well as pharmaceuticals for animals (i.e. veterinary applications). In a preferred aspect, the pharmaceutical is for human use and/or for animal husbandry.

The pharmaceutical can be for therapeutic purposes - which may be curative or palliative or preventative in nature. The pharmaceutical may even be for diagnostic purposes.

A pharmaceutically acceptable support may be for example a support in the form of compressed tablets, tablets, capsules, ointments, suppositories or drinkable solutions. Other suitable forms are provided below.

When used as - or in the preparation of - a pharmaceutical, the composition of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a solution or as a solid - depending on the use and/or the mode of application and/or the mode of administration.

The *Lactobacillus salivarius* strain Ls-33 (and any additional bacteria, if present) may be used according to the present invention as pharmaceutical ingredients. Here, the composition may be the sole active component, or it may be at least one of a number (i.e. 2 or more) of active components.

The pharmaceutical ingredient may be in the form of a solution or as a solid - depending on the use and/or the mode of application and/or the mode of administration.

The *Lactobacillus salivarius* strain Ls-33 (and any additional bacteria, if present) may be used according to the present invention in any suitable form - whether when alone or when present in a combination with other components or ingredients.

The *Lactobacillus salivarius* strain Ls-33 (and any additional bacteria, if present) may be used according to the present invention in the form of solid or liquid preparations or alternatives thereof. Examples of solid preparations include, but are not limited to tablets, capsules, dusts, granules and powders which may be wettable, spray-dried or freeze-dried. Examples of liquid preparations include, but are not limited to, aqueous, organic or aqueous-organic solutions, suspensions and emulsions.

Suitable examples of forms include one or more of: tablets, pills, capsules, ovules, solutions or suspensions, which may contain flavouring or colouring agents, for immediate-, delayed-, modified-, sustained-, pulsed- or controlled-release applications.

By way of example, if the composition of the present invention is used in a tablet form - such for use as a functional ingredient - the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethylcellulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerol, and combinations thereof.

The forms may also include gelatin capsules; fibre capsules, fibre tablets etc.; or even fibre beverages.

Further examples of form include creams. For some aspects the microorganism used in the present invention may be used in pharmaceutical and/or cosmetic creams such as sun creams and/or after-sun creams for example.

In one aspect, the composition according to the present invention may be administered in an aerosol, for example by way of a nasal spray, for instance for administration to the respiratory tract.

### Combinations

The composition of the present invention may additionally contain one or more prebiotics. Prebiotics are a category of functional food, defined as non-digestible food ingredients that beneficially affect the host by selectively stimulating the growth and/or activity of one or a limited number of bacteria (particularly, although not exclusively, probiotics, *Bifidobacteria* and/or lactic acid bacteria) in the colon, and thus improve host health. Typically, prebiotics are carbohydrates (such as oligosaccharides), but the definition does not preclude non-carbohydrates. The most prevalent forms of prebiotics are nutritionally classed as soluble fibre. To some extent, many forms of dietary fibre exhibit some level of prebiotic effect.

In one embodiment, a prebiotic is a selectively fermented ingredient that allows specific changes, both in the composition and/or activity in the gastrointestinal microflora that confers benefits upon host well-being and health.

Suitably, the prebiotic may be used according to the present invention in an amount of 0.01 to 100 g / day, preferably 0.1 to 50 g / day, more preferably 0.5 to 20 g / day.

Examples of dietary sources of prebiotics include soybeans, inulin sources (such as Jerusalem artichoke, jicama, and chicory root), raw oats, unrefined wheat, unrefined barley and yacon.

Examples of suitable prebiotics include alginate, xanthan, pectin, locust bean gum (LBG), inulin, guar gum, galacto-oligosaccharide (GOS), fructo-oligosaccharide (FOS), polydextrose (i.e. Litesse®), lactitol, lactosucrose, soybean oligosaccharides, isomaltulose (Palatinose™), isomalto-oligosaccharides, gluco-oligosaccharides, xylo-oligosaccharides, manno-oligosaccharides, beta-glucans, cellobiose, raffinose, gentiobiose, melibiose, xylobiose, cyclodextrins, isomaltose, trehalose, stachyose, panose, pullulan, verbascose, galactomannans, and all forms of resistant starches. A particularly preferred example of a prebiotic is polydextrose.

### Examples

### Example 1

### Materials and methods

C57BI/6J mice were obtained from Jackson Laboratories. One week before the mice were 3 months old, they were started on a high-fat diet (58 % of calories from fat) or normal fat diet *ad libitum* (Research Diets Inc.) (18% of calories from fat). At three months of age the mice started a 4-week treatment, which included a daily gavage with vehicle (saline) or *Lactobacillus salivarius* Ls33 (10⁹ CFU/day).

### Cardiac ischemia-reperfusion protocol

Following one month of treatment, mice were subjected to the cardiac ischemia-reperfusion protocol. Mice were anesthetized with an intraperitoneal injection of 250 mg/kg tribromoethanol, intubated and ventilated with 0.5-2.0% isoflurane. To maintain body temperature and restore potential loss of fluid, 500 µl of warmed sterile saline was injected into the dorsal subcutaneous space. The heart was exposed and the left coronary artery was visualized following a left anterior thoracotomy. The left coronary artery was occluded using 7-0 suture compressing a small piece of tubing (PE-10) to prevent vessel damage during occlusion. After 30 minutes of occlusion, the ligature was removed and the animal was allowed to recover.

During the occlusion of the left coronary artery, the heart suffers ischemia, which is then reperfused after 30 minutes. This reperfusion causes inflammation in the ischemic regions of the heart. Tissue samples from the ischemic and non-inschemic regions of the heart were collected to assess expressions of collagen (Col) III (Col3a1) and collagen (Col) I (Col1a2).

### Results

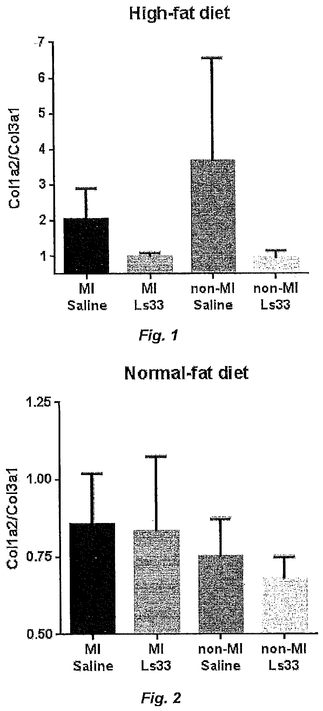

Figures 1 and 2 illustrate the results. Treatment with the probiotic Ls-33 decreased the Col I/Col III ratio in both ischemic (MI) and non-ischemic (non-MI) regions of the heart in mice on a high-fat diet (Fig. 1) and on a normal fat diet (Fig. 2). N = 3-5 per group.

The treatment with Ls-33 decreased the ratio of the stiff Col I to the more soft Col III in both ischemic and non-ischemic regions of the heart mainly on a high-fat diet (Figure 1), but slightly also on a normal fat diet (Figure 2), suggesting an improved remodeling process after myocardial infarction.

## Claims

1. A bacterium of the species *Lactobacillus salivarius* for use in treating cardiac dysfunction in a mammal, wherein said bacterium of the species *Lactobacillus salivarius* is strain 33 (Ls-33) (PTA-4800).

2. The bacterium for use of claim 1, wherein the cardiac dysfunction is selected from congestive heart failure (CHF), coronary artery disease, myocardial infarction, inflammatory heart disease and dilated cardiomyopathy.

3. The bacterium for use of claim 2, wherein the cardiac dysfunction is myocardial infarction.

4. The bacterium for use of claim 2, wherein the cardiac dysfunction is dilated cardiomyopathy.

5. The bacterium for use of claim 2, wherein the cardiac dysfunction is inflammatory heart disease.

6. The bacterium for use of claim 5, wherein the inflammatory heart disease is selected from endocarditis, inflammatory cardiomegaly and myocarditis.

7. The bacterium for use of any preceding claim, wherein the treatment of cardiac dysfunction comprises lowering of the collagen I (Col I) : collagen III (Col III) expression ratio.

8. The bacterium for use of any preceding claim, wherein the mammal in need of the treatment ingests a high-fat diet.

9. The bacterium for use of any preceding claim, wherein the mammal is a human.

10. The bacterium for use of any preceding claim, wherein the food product, dietary supplement or medicament additionally comprises one or more further probiotics.

11. The bacterium for use of any preceding claim, wherein the food product, dietary supplement or medicament additionally comprises a prebiotic.

12. The bacterium for use of claim 11, wherein the prebiotic is polydextrose.

13. The bacterium for use of any preceding claim, wherein the *Lactobacillus salivarius* is to be administered as a component of a food product, a dietary supplement or a pharmaceutical composition.

14. The bacterium for use of claim 13, wherein the *Lactobacillus salivarius* is to be administered as a component of a food product.

15. The bacterium for use of claim 14, wherein the *Lactobacillus salivarius* is to be administered as a component of a yogurt.

16. The bacterium for use of claim 13, wherein the *Lactobacillus salivarius* is to be administered as a component of a dietary supplement.

17. The bacterium for use of claim 13, wherein the *Lactobacillus salivarius* is to be administered as a component of a pharmaceutical composition.

18. The bacterium for use of any preceding claim, wherein the *Lactobacillus salivarius* is to be administered at a dosage of from 10⁶ to 10¹² CFU of bacteria/dose.

19. The bacterium for use of claim 18, wherein the *Lactobacillus salivarius* is to be administered at a dosage of 10⁹ to 10¹¹ CFU of bacteria/dose.

20. The bacterium for use of claim 15 wherein the yogurt contains from 10⁸ to 10¹² CFU of the bacteria per dose.

## Patentansprüche

1. Bakterium der Species *Lactobacillus salivarius* zur Verwendung in der Behandlung von kardialer Dysfunktion in einem Säuger, wobei das Bakterium der Species *Lactobacillus salivarius* Stamm 33 (Ls-33) (PTA-4800) ist.

2. Bakterium zur Verwendung nach Anspruch 1, wobei die kardiale Dysfunktion ausgewählt ist aus kongestiver Herzinsuffizienz (CHF, congestive heart failure), koronarer Arterienerkrankung, Myokardinfarkt, entzündlicher Herzerkrankung sowie dilatierter Kardiomyopathie.

3. Bakterium zur Verwendung nach Anspruch 2, wobei die kardiale Dysfunktion ein Myokardinfarkt ist.

4. Bakterium zur Verwendung nach Anspruch 2, wobei die kardiale Dysfunktion eine dilatierte Kardiomyopathie ist.

5. Bakterium zur Verwendung nach Anspruch 2, wobei die kardiale Dysfunktion eine entzündliche Herzerkrankung ist.

6. Bakterium zur Verwendung nach Anspruch 5, wobei die entzündliche Herzerkrankung ausgewählt ist aus Endokarditis, entzündlicher Kardiomegalie und Myokarditis.

7. Bakterium zur Verwendung nach einem der vorhergehenden Ansprüche, wobei die Behandlung von kardialer Dysfunktion Absenkung des Expressionsverhältnisses von Collagen I (Col I): Collagen III (Col III) umfasst.

8. Bakterium zur Verwendung nach einem der vorhergehenden Ansprüche, wobei der Säuger mit Bedarf für die Behandlung eine fettreiche Kost aufnimmt.

9. Bakterium zur Verwendung nach einem der vorhergehenden Ansprüche, wobei der Säuger ein Mensch ist.

10. Bakterium zur Verwendung nach einem der vorhergehenden Ansprüche, wobei das Nahrungsmittelprodukt, die Nahrungsmittelergänzung oder das Medikament zusätzlich ein Probiotikum oder mehrere Probiotika umfasst.

11. Bakterium zur Verwendung nach einem der vorhergehenden Ansprüche, wobei das Nahrungsmittelprodukt, die Nahrungsmittelergänzung oder das Medikament zusätzlich ein Probiotikum umfasst.

12. Bakterium zur Verwendung nach Anspruch 11, wobei das Probiotikum Polydextrose ist.

13. Bakterium zur Verwendung nach einem der vorhergehenden Ansprüche, wobei das *Lactobacillus salivarius* als eine Komponente eines Nahrungsmittelprodukts, einer Nahrungsmittelergänzung oder einer pharmazeutischen Zusammensetzung zu verabreichen ist.

14. Bakterium zur Verwendung nach Anspruch 13, wobei das *Lactobacillus salivarius* als eine Komponente eines Nahrungsmittelprodukts zu verabreichen ist.

15. Bakterium zur Verwendung nach Anspruch 14, wobei das *Lactobacillus salivarius* als eine Komponente eines Joghurts zu verabreichen ist.

16. Bakterium zur Verwendung nach Anspruch 13, wobei das *Lactobacillus salivarius* als eine Komponente einer Nahrungsmittelergänzung zu verabreichen ist.

17. Bakterium zur Verwendung nach Anspruch 13, wobei das *Lactobacillus salivarius* als eine Komponente einer pharmazeutischen Zusammensetzung zu verabreichen ist.

18. Bakterium zur Verwendung nach einem der vorhergehenden Ansprüche, wobei das *Lactobacillus salivarius* bei einer Dosierung von 10⁶ bis 10¹² CFU Bakterien/Dosis zu verabreichen ist.

19. Bakterium zur Verwendung nach Anspruch 18, wobei das *Lactobacillus salivarius* bei einer Dosierung von 10⁹ bis 10¹¹ CFU Bakterien/Dosis zu verabreichen ist.

20. Bakterium zur Verwendung nach Anspruch 15, wobei die Joghurt von 10⁸ bis 10¹² CFU Bakterien/Dosis enthält.

## Revendications

1. Bactérie de l'espèce *Lactobacillus salivarius* pour l'utilisation dans le traitement d'un dysfonctionnement cardiaque chez un mammifère, ladite bactérie de l'espèce *Lactobacillus salivarius* étant la souche 33 (Ls-33) (PTA-4800).

2. Bactérie pour l'utilisation selon la revendication 1, dans laquelle le dysfonctionnement cardiaque est choisi parmi une insuffisance cardiaque congestive (CHF), une maladie des artères coronaires, un infarctus du myocarde, une maladie cardiaque inflammatoire et une cardiomyopathie dilatée.

3. Bactérie pour l'utilisation selon la revendication 2, dans laquelle le dysfonctionnement cardiaque est un infarctus du myocarde.

4. Bactérie pour l'utilisation selon la revendication 2, dans laquelle le dysfonctionnement cardiaque est une cardiomyopathie dilatée.

5. Bactérie pour l'utilisation selon la revendication 2, dans laquelle le dysfonctionnement cardiaque est une maladie cardiaque inflammatoire.

6. Bactérie pour l'utilisation selon la revendication 5, dans laquelle la maladie cardiaque inflammatoire est choisie parmi une endocardite, une cardiomégalie inflammatoire et une myocardite.

7. Bactérie pour l'utilisation selon l'une quelconque des revendications précédentes, dans laquelle le traitement d'un dysfonctionnement cardiaque comprend l'abaissement du rapport d'expression collagène I (Col I) : collagène III (Col III).

8. Bactérie pour l'utilisation selon l'une quelconque des revendications précédentes, dans laquelle le mammifère ayant besoin du traitement ingère un régime riche en matières grasses.

9. Bactérie pour l'utilisation selon l'une quelconque des revendications précédentes, dans laquelle le mammifère est un être humain.

10. Bactérie pour l'utilisation selon l'une quelconque des revendications précédentes, dans laquelle le produit alimentaire, le complément alimentaire ou le médicament comprend de plus un ou plusieurs probiotiques.

11. Bactérie pour l'utilisation selon l'une quelconque des revendications précédentes, dans laquelle le produit alimentaire, le complément alimentaire ou le médicament comprend de plus un prébiotique.

12. Bactérie pour l'utilisation selon la revendication 11, dans laquelle le prébiotique est un polydextrose.

13. Bactérie pour l'utilisation selon l'une quelconque des revendications précédentes, dans laquelle le *Lactobacillus salivarius* doit être administré en tant que constituant d'un produit alimentaire, d'un complément alimentaire ou d'une composition pharmaceutique.

14. Bactérie pour l'utilisation selon la revendication 13, dans laquelle le *Lactobacillus salivarius* doit être administré en tant que constituant d'un produit alimentaire.

15. Bactérie pour l'utilisation selon la revendication 14, dans laquelle le *Lactobacillus salivarius* doit être administré en tant que constituant d'un yaourt.

16. Bactérie pour l'utilisation selon la revendication 13, dans laquelle le *Lactobacillus salivarius* doit être administré en tant que constituant d'un complément alimentaire.

17. Bactérie pour l'utilisation selon la revendication 13, dans laquelle le *Lactobacillus salivarius* doit être administré en tant que constituant d'une composition pharmaceutique.

18. Bactérie pour l'utilisation selon l'une quelconque des revendications précédentes, dans laquelle le *Lactobacillus salivarius* doit être administré à une posologie de 10⁶ à 10¹² UFC de bactéries/dose.

19. Bactérie pour l'utilisation selon la revendication 18, dans laquelle le *Lactobacillus salivarius* doit être administré à une posologie de 10⁹ à 10¹¹ UFC de bactéries/dose.

20. Bactérie pour l'utilisation selon la revendication 15, dans laquelle le yaourt contient de 10⁸ à 10¹² UFC des bactéries par dose.
